# EUROPÄISCHE PATENTANMELDUNG

(11) **EP 2 689 799 A1**
(43) Veröffentlichungstag der Anmeldung: **29.01.2014**
(21) Anmeldenummer: 13003498.6
(22) Anmeldetag: 11.07.2013
(51) Int. Cl.: A61N 1/04, A61N 1/20, A61M 37/00, A61N 1/05, A61N 1/32, A61B 18/14, A61H 15/00

(54) **Vorrichtung zur Behandlung der Haut**

(30) Priorität: 24.07.2012 DE 102012014653
(71) Anmelder: Liebl, Edelgard, 67860 Zelsheim/Elsass (FR)
(72) Erfinder: Liebl, Horst, 67860 Zelsheim (FR)
(74) Vertreter: Tomerius, Isabel

(57) **Zusammenfassung**

Die Erfindung betrifft eine Vorrichtung (1) zur Behandlung der Haut (2) mit einer um ihre Längsachse (30) drehbar gelagerten Walze (3), auf deren Außenumfangsfläche (31) eine Vielzahl von Nadeln (4) radial nach außen vorsteht. Sie weist zudem Mittel (5) zum elektrischen Kontaktieren wenigstens eines Teils der Nadeln (4) mit einer Spannungsquelle (6) auf, umfassend
- einen ersten, mit einem ersten Pol der Spannungsquelle (6) verbindbaren Anschluss (50),
- einen zweiten, mit einem zweiten Pol der Spannungsquelle (6) verbindbaren Anschluss (51),
- einen ersten Kontaktbereich (52), der elektrisch leitend mit ersten Nadeln (40) verbunden ist,
- einen zweiten, vom ersten Kontaktbereich (52) räumlich getrennten und elektrisch isolierten

zweiten Kontaktbereich (53), der elektrisch leitend mit zweiten Nadeln (41) verbunden ist, wobei die Anschlüsse (50, 51) und die Kontaktbereiche (52, 53) so ausgebildet sind, dass bei einer Verbindung von erstem Anschluss (50) und erstem Kontaktbereich (52) auch der zweite Anschluss (51) mit dem zweiten Kontaktbereich (53) verbunden ist und die ersten Nadeln (40) und die zweiten Nadeln (41) jeweils in einer Reihe angeordnet und die Reihen in Umfangsrichtung der Walze (3) voneinander beabstandet sind.

## Beschreibung

Die Erfindung betrifft eine Vorrichtung zur Behandlung der Haut, welche eine um ihre Längsachse drehbar gelagerte Walze umfasst, über deren Außenumfangsfläche eine Vielzahl von Nadeln radial nach außen vorsteht, und die sich des Anlegens einer Spannung an die Haut, beispielsweise zur Förderung der Wundheilung, bedient.

Ohne näher auf die Ätiologie chronischer bzw. nicht heilender Wunden einzugehen, steht fest, dass die Hauptursache verzögerter oder ganz aussetzender Heilung eine mangelhafte Durchblutung im Wundgebiet ist. Die Wunde wird nur noch unzureichend oder nicht mehr mit Sauerstoff und Nährstoffen versorgt. Hinzu kommen weitere Ursachen bakteriellen und/oder toxischen Ursprungs, die die Proliferation von wundreinigenden und wundaufbauenden Zellen hemmen oder gar ganz unterdrücken. Alle Ursachen führen zum Zusammenbruch von Zellinformationen auf chemo- und/oder elektrotaxischem Weg und verhindern die Proliferation von Zellen zum Aufbau von neuem Gewebe. Bei jeder Wunde, akut oder chronisch, kommt es zu einem Kollaps der bioelektrischen Spannungen (Trans Epitheliale Potentiale - TEPs (Foulds and Barker [1, 19], siehe "Literatur"), kurz: zu einem Kurzschluss derselben (Kloth [2]). Sehr vermutlich verhindert zusätzlich ein Mangel, eine pH-Verschiebung oder ein Austrocknen des interstitiellen Elektrolyts [3, 4] die Aktivität der lonenpumpe der Zellen in der Wundumgebung. Diese wird immer nach einer Verletzung aktiviert und dient zum Aufbau eines natürlichen elektromagnetischen Feldes, zur Genexpression von transformierenden Wachstumsfaktoren (Transforming Growth Factor (TGF)) und Proliferation von Fibroblasten und anderen Zellen. Diese Mangelerscheinungen unterdrücken den Aufbau eines natürlichen elektromagnetischen Feldes [5].

Die wichtigsten Voraussetzungen für das Management chronischer Wunden bestehen aus vier Komponenten: a) Gewebemanagement, b) Entzündungs- und Infektionskontrolle, c) Feuchtigkeitsbalance und d) Epithelisierung. In vielen Fällen kommt es trotzdem zu einem Stillstand des Wundheilungsverlaufs, oft zu einer Schließung der Wunde, aber in vielen Fällen auch zu einer unvermeidlichen Amputation der betroffenen Extremitäten.
Theoretische Überlegungen und ihre in die Praxis umgesetzten Messungen, Geräte und Vorrichtungen haben bewiesen, dass chronische Wunden positiv mit Elektrostimulation beeinflusst werden können, und zwar so weit, dass dies wieder zu einem Wundverschluss führt. Falanga et al. [6] haben in vitro gezeigt, dass die Elektrostimulation die Rezeptoren von TGF-β auf humanen Haut-Fibroblasten, im Vergleich zu Kontrollfibroblasten, um den Faktor sechs hochregulieren. Andere Untersuchungen haben ergeben, dass Zellen, die an der Wundheilung beteiligt sind, zur Anode oder Kathode eines elektrischen Feldes wandern (Galvanotaxis) [7 - 17]. Sehr vermutlich reagieren Zellen auf elektrische Felder durch elektrophoretische Bewegung von Proteinen innerhalb der Plasmamembran [18]. Ebenso wird die Migration von Keratinozyten durch elektrische Felder beeinflusst [14, 15]. Wie Rowley [19] und Wolcott et al. [20] nachgewiesen haben, hat ein Gleichstrom-elektrisches Feld einen bakteriostatischen Effekt. Konsequenterweise beschäftigen sich andere Forscher mit silberbeschichteten Anoden [21 - 28] und konnten in vitro und in vivo belegen, dass Silber-Kationen einen bakteriostatischen und antibakteriellen Effekt haben. Junger et al. [29] berichteten über eine verbesserte Durchblutung durch Zunahme der Kapillardichte durch elektrische Felder. Bei 15 Patienten mit Beingeschwüren, nach mehrmonatiger Standartbehandlung, verbesserten sich die Geschwüre durch Anlegen eines elektrischen Feldes um durchschnittlich 43,5 %.

Der Nachteil aller bisher eingesetzten Wundbehandlungs-Vorrichtungen mit positiver und negativer Elektrode besteht darin, dass sie keine genau definierten Ströme an und in der Wunde generieren können. Die Ursachen sind vielfältig und sollen hier nur kurz skizziert werden:
- schwankendes feuchtes Milieu auf und in der Wunde,
- unterschiedlich entfernte Platzierung der (Gegen-) Elektrode, gegenüber der Elektrode auf der Wunde,
- beeinflussbarer elektrischer Widerstand durch wechselnde Hautfeuchte unter der Gegenelektrode,
- chronische Wunden (wenn auch selten) oberhalb des Herzmeridians können aufgrund der möglichen Beeinflussung von Herz- und Hirnströmen nicht behandelt werden.
Aufgrund der beschriebenen Probleme weisen die Vorrichtungen des Standes der Technik den Nachteil auf, dass sie häufig keine zufriedenstellenden und reproduzierbaren Ergebnisse bei der Wundbehandlung erzielen.

**Aufgabe** der Erfindung ist es deshalb, eine Vorrichtung zur Behandlung der Haut anzugeben, welche es ermöglicht, gezielt und in reproduzierbarer Weise ein elektrisches Feld an der zu behandelnden Haut anzulegen und so einen lonenfluss zu induzieren.

Die **Lösung** gelingt mit der Vorrichtung gemäß Anspruch 1. Bevorzugte Weiterbildungen sind in den Unteransprüchen beschrieben. Demnach betrifft die Erfindung eine Vorrichtung zur Behandlung der Haut mit einer um ihre Längsachse drehbar gelagerten Walze, auf deren Außenumfangsfläche eine Vielzahl von Nadeln radial nach außen vorsteht. Derartige Vorrichtungen, deren mit Mikronadeln besetzte Walze über die zu behandelnde Haut gerollt wird, sind grundsätzlich bekannt und in der DE 10063634 A1 der Anmelderin beschrieben. Für den Grundaufbau einer solchen Vorrichtung kann auch auf die EP 1764129 A1 der Anmelderin verwiesen werden. Die dort beschriebene Vorrichtung weist mehrere mit Nadeln besetzte Walzen auf. Die beim Drehen der Walze(n) in die Haut eindringenden Nadeln stimulieren die Collagenese und Angiogenese und fördern bereits ohne Gabe von Wirkstoffen die Regeneration der Haut und die Wundheilung. Außerdem können hypertrophe Narben abgebaut und eingesunkene Narben aufgebaut werden. Durch die temporäre Bildung feiner Stichkanäle kann zudem die Aufnahme von Wirkstoffen in der Dermis durch leichtere Überwindung der Epidermis-Barriere erheblich verbessert werden.

Mit der vorliegenden Erfindung wurden die oben beschriebenen Vorrichtungen weitergebildet, indem diese mit Mitteln zum elektrischen Kontaktieren wenigstens eines Teils der Nadeln mit einer Spannungsquelle versehen wurden. Diese Mittel umfassen:
- einen ersten, mit einem ersten Pol der Spannungsquelle verbindbaren Anschluss,
- einen zweiten, mit einem zweiten Pol der Spannungsquelle verbindbaren Anschluss,
- einen ersten Kontaktbereich, der elektrisch leitend mit ersten Nadeln verbunden ist,
- einen zweiten, vom ersten Kontaktbereich räumlich getrennten und elektrisch isolierten zweiten
Kontaktbereich, der elektrisch leitend mit zweiten Nadeln verbunden ist, wobei die Anschlüsse und die Kontaktbereiche so ausgebildet sind, dass bei einer Verbindung von erstem Anschluss und erstem Kontaktbereich auch der zweite Anschluss mit dem zweiten Kontaktbereich verbunden ist. Dabei sind die ersten Nadeln und die zweiten Nadeln jeweils in einer Reihe angeordnet und die Reihen in Umfangsrichtung der Walze voneinander beabstandet.

Bei der erfindungsgemäßen Vorrichtung zur Behandlung der Haut - nachfolgend vereinfachend als "Nadelroller" bezeichnet - werden also erste und zweite Nadeln mit den unterschiedlichen Polen einer Spannungsquelle verbunden. Erste Nadeln sind dabei in einer (ersten) Reihe angeordnet, die sich in Umfangsrichtung der Walze beabstandet zu der (zweiten) Reihe der zweiten Nadeln befindet. Durch die Beabstandung von erster und zweiter Reihe von Nadeln in Umfangsrichtung der Walze wird aufgrund der Verbindung mit den unterschiedlichen Polen der Spannungsquelle zwischen den unterschiedlich gepolten Nadelspitzen, wenn diese in die Haut eingedrungen sind, ein elektrisches Feld erzeugt. Welcher Pol an welche Nadelreihe angeschlossen wird, spielt grundsätzlich keine Rolle. Es muss lediglich gewährleistet sein, dass die beiden Nadelreihen, wenn der Nadelroller über die Haut bewegt wird, gemeinsam in die Haut eindringen können. Bezogen auf die Anordnung entlang der Außenumfangsfläche der Walze, über die die Nadeln vorstehen, ist die Reihe erster Nadeln daher bevorzugt in der Umfangsrichtung zu der Reihe der zweiten Nadeln unmittelbar benachbart angeordnet. Auf diese Weise kann sichergestellt werden, dass der Abstand der ersten Nadeln von den zweiten Nadeln gering genug ist, dass beide Nadelreihen gemeinsam in die Haut eindringen können. Zweckmäßig verlaufen die Nadelreihen außerdem parallel zur Längsachse der Walze und parallel zueinander, aber auch leicht schräg verlaufende, gegenüber der Längsachse gekippte Reihen sind grundsätzlich möglich.

Bevorzugt sind die Nadeln einer Reihe exakt linear hintereinander in Längsrichtung der Walze angeordnet, besonders bevorzugt über die gesamte Breite der Walze. Dies ist aber nicht zwingend, sondern es ist auch möglich, die Reihen kürzer zu machen und/oder die Nadeln einer Reihe gegeneinander zu versetzen, solange es nicht zu einem Ineinandergreifen verschiedener Nadelreihen kommt. Erste und zweite Nadeln können also nicht nur jeweils linear hintereinander, sondern auch innerhalb eines entlang der Breite der Walze verlaufenden Streifens angeordnet sein, wobei die Längskanten der Streifen durch die am weitesten außen liegenden Nadeln einer Reihe definiert werden und wobei der die erste Nadelreihe umfassende Streifen und der die zweite Nadelreihe umfassende Streifen dann einen Abstand zueinander aufweisen. Dringen die Nadeln der ersten und der zweiten Nadelreihe in die Haut ein, kommt es aufgrund der Feuchtigkeit der Haut zu einem lonenfluss in der Haut von den mit dem Pluspol der Spannungsquelle verbundenen Nadelenden in Richtung auf die Nadelenden, welche mit dem Minuspol der Spannungsquelle verbunden sind. Das elektromagnetische Feld baut sich dabei ausschließlich und gezielt zwischen den zwei unterschiedlich gepolten Nadelreihen auf und kann durch geeignete Wahl der Nadellänge und des Reihenabstandes millimetergenau eingestellt werden.

Die erfindungsgemäße Vorrichtung erlaubt also die gezielte Elektrostimulation der Haut und kombiniert die auf diese Weise erzielbaren positiven Wirkungen in idealer Weise mit den hautstimulierenden Wirkungen eines konventionellen Nadelrollers. Das in der Haut induzierte elektromagnetische Feld ersetzt das nach einer Verletzung nicht mehr existente natürliche elektromagnetische Feld, vergleichbar dem Einsatz eines Defibrillators nach einem Herzstillstand, und regt den lonenfluss in der Haut wieder an. So wird einem Kollaps der bioelektrischen Spannungen (TEP) entgegengewirkt, die Blut- und Sauerstoffversorgung der Wunde kommt wieder in Gang und damit die Versorgung der Wunde mit den zur Wundheilung benötigten körpereigenen Stoffen. Insgesamt wird somit die Wundheilung erheblich beschleunigt und befördert und gleichzeitig das Risiko einer Vernarbung der abheilenden Wunde verringert.

Eine weitere Anwendungsmöglichkeit der erfindungsgemäßen Vorrichtung liegt in der Behandlung alternder Haut. Eine Ursache der Hautalterung liegt darin, dass die periphere Durchblutung mit zunehmenden Alter abnimmt. Durch Unterversorgung mit Sauerstoff, Abnahme der Zellernährung und verminderte Entschlackung kommt es so zur Hautalterung. Zudem verringert sich die Teilungsfähigkeit der Hautzellen, insbesondere der Keratinozyten, beispielsweise in Folge intensiver Sonnenbestrahlung und Einwirkung von UVA- bzw. UVB-Strahlung, was sich ebenfalls negativ auf das Aussehen der Haut auswirkt. Auch diese im Alter nachlassenden Erneuerungsprozesse können durch Behandlung mit der erfindungsgemäßen Vorrichtung gefördert werden, wobei die Einwirkung eines elektromagnetischen Feldes die Zellregeneration wieder aktiviert.

Innerhalb der Reihen erster und zweiter Nadeln müssen nicht sämtliche der Nadeln mit der Spannungsquelle verbunden werden. Es können vielmehr innerhalb einer Nadelreihe selektiv bestimmte Nadeln für die Kontaktierung mit der Spannungsquelle ausgewählt werden. Bevorzugt erfolgt die elektrische Kontaktierung der Nadeln mit den Polen der Spannungsquelle dabei so, dass in dem Hautbereich, der von den mit Spannung beaufschlagten Nadeln penetriert wird, ein elektromagnetisches Feld erzeugt wird, in dem ein lonenfluss in einer bestimmten Richtung vorherrscht. Diese Richtung sollte sich nach Möglichkeit im Verlaufe der Hautbehandlung mit der erfindungsgemäßen Vorrichtung nicht oder nicht wesentlich ändern und sich möglichst nicht umkehren, da dies die Wundheilung beeinträchtigen oder zumindest verzögern könnte. Bevorzugt weist der Nadelroller zudem mehrere erste und zweite Nadelreihen auf. Durch abwechselnde Polung benachbarter Nadelreihen ist es möglich, einen lonenfluss im behandelten Hautbereich in oder entgegen der Fortbewegungsrichtung des Nadelrollers über die Haut zu erzeugen. Hierzu muss allerdings sichergestellt werden, dass lediglich Nadelreihenpaare mit gleichbleibend unterschiedlicher Polung- zum Beispiel immer erste Reihe in Fortbewegungsrichtung positiv gepolt, zweite Nadelreihe negativ gepolt - gleichzeitig in die Haut eindringen und die Haut nicht von weiteren Nadelreihen unterschiedlicher Polarität durchdrungen wird, welche den lonenfluss in der Haut umkehren könnten. Dies lässt sich grundsätzlich auf unterschiedliche Weise sicherstellen.

In einer besonders einfachen, wenn auch nicht bevorzugten Variante, werden lediglich zwei benachbarte Reihen von Nadeln entlang der Außenumfangsfläche mit den Polen der Spannungsquelle verbunden, nicht jedoch die übrigen über die Außenumfangsfläche der Walzen vorstehenden Nadeln. Auf diese Weise lässt sich jedoch nur ein geringes und eng begrenztes elektromagnetisches Feld in der Haut erzeugen. Eine weitere Möglichkeit besteht darin, durch gezielte Einstellung der Nadellängen und Abstände der Nadelreihen zueinander sicherzustellen, dass jeweils nur die Nadeln zweier benachbarter Reihen gleichbleibender Polungspaarung in die Haut eindringen können, nicht jedoch außerhalb dieser beiden Reihen liegende Nadelreihenpaare. Auf diese Weise lassen sich grundsätzlich sämtliche Reihen Nadeln, die über die Walze vorstehen, mit der Spannungsquelle verbinden. Nachteilig kann hier jedoch sein, dass die zueinander benachbarten Nadelreihenpaare relativ weit auseinander liegen müssen, so dass sich die Gesamtzahl der über die Außenumfangsfläche der Walze vorstehenden Nadeln verringert und sich entsprechend die Wirkung des Nadelrollers dadurch abschwächt und, anders gesagt, zur Erzielung der gewünschten Wirkung eine längere Behandlungsdauer erforderlich wird. Außerdem sind die unregelmäßigen Abstände in Umfangsrichtung (geringerer Abstand zwischen den Reihen innerhalb eines Nadelreihenpaares, größerer Abstand zwischen benachbarten Reihenpaaren) bei der Fortbewegung des Nadelrollers über die Haut unter Umständen nachteilig.

Eine weitere Variante besteht darin, dass es über die Außenumfangsfläche der Walze vorstehend mehrere Paare von Reihen erster Nadeln und zweiter Nadeln gibt, die jeweils entweder mit dem ersten oder dem zweiten Pol der Spannungsquelle verbunden sind. Diese mit den unterschiedlichen Polen der Spannungsquelle verbundenen Paare von Nadelreihen werden jedoch jeweils von wenigstens einer Reihe von Nadeln unterbrochen, die nicht mit der Spannungsquelle verbunden sind. Selbst wenn also die Nadeln dieser nicht mit Spannung beaufschlagten Reihen gleichzeitig mit denjenigen Nadelreihen, die mit der Spannungsquelle in Verbindung sind, in die Haut eindringen, beeinflusst dies den lonenfluss innerhalb der Haut nicht. Bei gleichbleibendem Anschluss der in Umfangsrichtung aufeinander folgenden Nadelreihen (zum Beispiel Plus, Minus, Null, Plus, Minus, Null etc.) an die unterschiedlichen Pole bleibt die Richtung des elektromagnetischen Feldes in der Haut immer konstant. Dabei versteht es sich von selbst, dass innerhalb einer Reihe von Nadeln nicht sämtliche Nadeln mit dem Pol einer Spannungsquelle verbunden sein müssen, sondern auch nur ein Teil derselben verbunden sein kann, beispielsweise jede zweite Nadel. Dies gilt grundsätzlich für alle Beispiele, in denen Reihen von Nadeln mit der Spannungsquelle kontaktiert werden.

In einer besonders bevorzugten Ausführungsform der Erfindung sind erster Anschluss, zweiter Anschluss, erster Kontaktbereich und zweiter Kontaktbereich, mit denen die ersten Nadel und die zweiten Nadeln mit den Polen der Spannungsquelle verbunden werden können, so ausgelegt, dass jeweils nur diejenige ersten und zweiten Nadeln mit der Spannungsquelle verbunden sind, welche gerade die Haut penetrieren. Dies kann dadurch erreicht werden, dass erster Anschluss und erster Kontaktbereich sowie zweiter Anschluss und zweiter Kontaktbereich jeweils lösbar miteinander verbunden sind. Eine elektrische Kontaktierung erfolgt jeweils nur dann, wenn die entsprechenden Nadeln durch Rotation der Walze in eine Position gebracht worden sind, in welcher sie in die Haut eindringen können. Besonders bevorzugt ist es daher, wenn die Kontaktierung zwischen dem jeweils zusammengehörigen Paar von Anschluss und Kontaktbereich durch die Rotation der Walze um ihre Längsachse herstellbar und auch wieder trennbar ist. Realisierbar ist dies beispielsweise, wenn die Anschlüsse und Kontaktbereiche als Schleifkontakte ausgebildet sind. Konkret ist dabei der mit dem ersten Pol der Spannungsquelle verbindbare erste Anschluss derart relativ zu dem ersten Kontaktbereich orientiert, der mit den ersten Nadeln elektrisch verbunden ist, dass die beiden Kontaktflächen aufeinander zu liegen kommen, wenn die Walze so über die Haut gerollt wird, dass die ersten Nadeln in Richtung auf die Haut zeigen. Gleiches gilt im Hinblick auf zweiten Anschluss, zweiten Kontaktbereich und die mit diesem elektrisch verbundenen zweiten Nadeln. Sobald also erste und zweite Nadeln in die Haut eindringen, kommen die jeweils zusammengehörenden Schleifkontakte aufeinander zu liegen, es beginnt Strom zu fließen, und zwischen den ersten Nadeln und den zweiten Nadeln bildet sich ein elektromagnetisches Feld aus, das wiederum einen lonenfluss zwischen ersten und zweiten Nadeln in der Haut induziert. Durch Weiterdrehen der Walze werden die ersten und zweiten Nadeln wieder aus der Haut herausgezogen, wobei sich die Schleifkontakte voneinander lösen und der Stromfluss unterbrochen wird. Diese Art der Kontaktierung erlaubt den Anschluss sämtlicher Nadelreihen in Umfangsrichtung der Walze, selbst wenn diese relativ eng nebeneinander angeordnet sind, ohne dass es zu unbeabsichtigten Richtungswechseln des in der Haut induzierten elektromagnetischen Feldes kommt, wenn mehr als zwei benachbarte Nadelreihen in die Haut eindringen.

Erster und zweiter Kontaktbereich, die zur Kontaktierung der mit der Spannungsquelle verbundenen Anschlüsse mit den ersten und zweiten Nadeln dienen, können grundsätzlich an beliebiger Stelle zu einer Oberfläche der Walze herausgeführt sein, um die Kontaktierung zu ermöglichen. Besonders bevorzugt sind erster und zweiter Kontaktbereich an einer Stirnseite der Walze angeordnet, und bevorzugt befinden sie sich auf gegenüberliegenden Stirnseiten der Walze. Auf diese Weise sind die beiden Kontaktbereiche räumlich sehr weit voneinander getrennt, und es steht für beide hinreichend Platz für die Kontaktierung zur Verfügung. Erster und zweiter Anschluss, die der elektrischen Verbindung der Kontaktbereiche mit der Spannungsquelle dienen, sind entsprechend den Positionen der zugehörigen Kontaktbereiche angeordnet. Befinden sich letztere an einer Stirnseite der Walze, ist es bevorzugt, die Anschlüsse an einem innenseitigen Ende einer die Walze haltenden Gabel anzuordnen. Befinden sich erster und zweiter Kontaktbereich an gegenüberliegenden Stirnseiten der Walze, liegen erster und zweiter Anschluss entsprechend an gegenüberliegenden innenseitigen Enden der Gabel. Um für einen sicheren Kontakt zwischen den zusammengehörigen Paaren von Anschluss und Kontaktbereich zu sorgen, kann wenigstens eines der beiden Paarungsteile in Richtung auf das andere federnd gelagert sein.

Im Falle der reihenweisen Anordnung von Nadeln, wie sie vorstehend beschrieben wurde, ist es bevorzugt, dass mehrere oder alle ersten Nadeln in Reihe mit dem ersten Kontaktbereich verbunden sind und mehrere oder alle zweiten Nadeln in Reihe mit dem zweiten Kontaktbereich. Besonders bevorzugt ist es dabei, wenn jede der kontaktierbaren Nadelreihen sowohl einen ersten Kontaktbereich zur Kontaktierung des ersten Anschlusses als auch einen zweiten Kontaktbereich zur Kontaktierung des zweiten Anschlusses aufweist. Je nach der Position der Nadelreihe im Bezug auf den ersten und den zweiten Anschluss kann deshalb die Nadelreihe einerseits als erste Nadelreihe fungieren, wenn sie mit dem ersten Pol der Spannungsquelle kontaktiert wird, oder als zweite Nadelreihe, wenn die Kontaktierung mit dem zweiten Pol der Spannungsquelle erfolgt. Abhängig von ihrer Position, d. h. der Rotation der Walze, kann eine Nadelreihe demnach entweder mit dem positiven oder dem negativen Pol der Spannungsquelle verbunden werden. Mit der Rotation der Walze wechselt also die Polarität der Nadeln einer Nadelreihe beispielsweise von positiv auf negativ und anschließend auf gar nicht geladen.

Die Kontaktierung der elektrisch leitend miteinander verbundenen Teile der erfindungsgemäßen Vorrichtung kann grundsätzlich auf jede beliebige mögliche Weise erfolgen, beispielsweise mithilfe elektrisch leitender Kabel. Diese Art der Kontaktierung wird zweckmäßig bei der elektrischen Verbindung von erstem und zweitem Anschluss mit den Polen der Spannungsquelle eingesetzt, wobei es bevorzugt ist, die Kabel im Inneren der Gabel zu verlegen und aus dem hinteren Ende des Gabelgriffes herauszuführen, wenn als Spannungsquelle eine externe Spannungsquelle, die außerhalb des Nadelrollers befindlich ist, verwendet wird. Es ist grundsätzlich jedoch ebenfalls möglich, die Spannungsquelle in den Nadelroller zu integrieren, beispielsweise in Form einer Batterie oder eines Akkus. In diesem Fall können die Reihen von Nadeln auch unmittelbar mit den Polen der Spannungsquelle verbunden werden. Als Kontaktbereiche dienen dann beispielsweise die Fußpunkte (die von den über die Walze vorstehenden Nadelspitzen abgelegenen Nadelenden) der jeweiligen Nadeln. Die Kontaktbereiche sind in diesem Fall integrale Bestandteile der Nadeln; für die elektrische Kontaktierung der Kontaktbereiche mit den Nadeln ist kein gesondertes Verbindungsmittel notwendig. Zweckmäßig ist für diesen Fall einer integrierten Spannungsquelle, die bevorzugt im Inneren der Walze angeordnet wird, ein Schalter zum Zu- und Abschalten der Spannungsquelle vorgesehen. Im Falle einer nicht wieder aufladbaren Spannungsquelle handelt es sich bei der erfindungsgemäßen Vorrichtung um ein Wegwerfprodukt, das nach dem Aufbrauchen der in der Spannungsquelle gespeicherten Energie entsorgt wird.

Bevorzugt sind allerdings solche Vorrichtungen, bei denen die Spannungsquelle als externe Spannungsquelle ausgebildet ist, welche sich außerhalb des Nadelrollers befindet. Bevorzugt handelt es sich um eine Gleichspannungsquelle, die entweder linearen und/oder gepulsten Gleichstrom zuführen kann. Besonders zweckmäßig ist die Spannungsquelle als variable Spannungsquelle ausgebildet, die eine Einstellung der zugeführten Spannung erlaubt. Dabei können beispielsweise die Größe der zugeführten Spannung, die Größe des zugeführten Stroms, die Art des zugeführten Stroms (gepulst oder linear) sowie die Polung von der Bedienperson gezielt eingestellt werden. Besonders bevorzugt ist die Spannungsquelle so ausgebildet, dass sie eine Gleichspannung im zwei- bis vierstelligen, insbesondere dreistelligen Millivolt-Bereich zuführen kann. Die Stromstärke wird üblicherweise im Milliampere-Bereich liegen.

Für die Kontaktierung von erstem Kontaktbereich und ersten Nadeln sowie zweitem Kontaktbereich und zweiten Nadeln können ebenfalls beliebige elektrische Leiter wie beispielsweise Kabel eingesetzt werden. Hier ist es jedoch bevorzugt, die elektrische Kontaktierung mit Hilfe elektrisch leitfähiger Kunststoffe herzustellen. Bei dem elektrisch leitfähigen Kunststoff kann es sich auch um einen leitfähigen Klebstoff handeln, der gleichzeitig zur Befestigung der Nadeln in der Walze dienen kann. Besonders bevorzugt werden dabei zumindest die von der Umfangsfläche der Walze abgelegenen hinteren Enden der Nadeln in den leitfähigen Kunststoff eingebettet.

Von den herkömmlichen "nicht-elektrischen" Nadelrollern ist es bekannt, die Nadeln in Nadelträgern aus Kunststoff einzubetten. Verwendet werden hierbei beispielsweise Nadelträger, welche in einem Querschnitt senkrecht zur Längsachse der Walze im Wesentlichen tortenstückförmig ausgebildet sind und jeweils eine in Breitenrichtung der Walze, parallel zur Walzenlängsachse, verlaufende Reihe von Nadeln halten. Mehrere solcher tortenstückförmigen Nadelträger werden dann miteinander verbunden, um die vollständige Walze zu ergeben. Derartige tortenstückförmige Nadelträger können bei der Herstellung der erfindungsgemäßen elektrifizierten Vorrichtung ebenfalls eingesetzt werden. Bevorzugt bestehen die Nadelträger dann zumindest teilweise aus einem leitfähigen Kunststoff, der die einzelnen Nadeln elektrisch miteinander kontaktiert. Um Kurzschlüsse zwischen den einzelnen Nadelträgern und den in sie eingebetteten Nadeln zu verhindern, müssen die jeweiligen Nadelträger nun jedoch elektrisch gegeneinander isoliert werden. Bevorzugt geschieht dies dadurch, dass die Nadelträger in einen Träger eingesetzt werden, der aus einem nichtleitenden Material, insbesondere einem nichtleitenden Kunststoff, besteht, der so aufgebaut ist, dass er einen unmittelbaren Kontakt von Nadelträger zu Nadelträger verhindert. Zweckmäßig weist dieser Träger daher einen in Richtung senkrecht zur Walzenlängsachse sternförmigen Querschnitt auf. Zwischen die einzelnen Strahlen des Sterns werden die Nadelträger eingesetzt. Besonders bevorzugt geschieht dies derart, dass die Nadelträger allein durch einen Klemmsitz in dem Träger gehalten werden und nicht mehr gesondert befestigt werden müssen. Alternativ oder zusätzlich können die Nadelträger jedoch auch in den Träger eingeklebt werden.

Bestehen die Nadelträger insgesamt aus einem elektrisch leitfähigen Kunststoff, kann es zweckmäßig sein, deren mit der Haut in Kontakt kommende Oberflächen elektrisch zu isolieren. Dies kann beispielsweise dadurch erfolgen, dass auf der die Außenumfangsfläche der Walze bildenden Oberfläche des Nadelträger eine nichtleitende Beschichtung aufgebracht wird. Hierbei kann es sich auch um einen Überzug aus einer nichtleitenden Kunststofffolie, beispielsweise aus einer Schrumpffolie handeln. Falls gewünscht, können auch die unmittelbar an die Außenumfangsfläche der Walze angrenzenden vorstehenden Bereiche der Nadeln elektrisch isoliert werden, beispielsweise ebenfalls mittels einer nichtleitenden Beschichtung, so dass lediglich die äußersten Nadelspitzen elektrisch leitend sind und sich nur in diesen Bereichen das elektromagnetische Feld in der Haut ausbilden kann. Zum Schutz und zur Verhinderung allergischer Reaktionen können die Nadeln zumindest in den Bereichen, mit denen sie in die Haut eindringen, beschichtet werden. Die Schutzschicht kann zum Beispiel aus Gold, Silber, Titan oder anderen inerten Metallen sowie deren Mischungen oder Legierungen bestehen.

Wie bereits erwähnt, kann die erfindungsgemäße Vorrichtung von ihrem Grundaufbau im Wesentlichen dem in der DE 10063634 A1 beschriebenen Nadelroller entsprechen. Bei Anwendung der Erfindung auf die in der EP 1764129 A1 beschriebene Vorrichtung können eine oder mehrere der Walzen mit dem Mittel zum elektrischen Kontaktieren versehen werden. Da der Grundaufbau der Nadelroller im Prinzip bekannt ist, muss auf diesen hier nicht weiter eingegangen werden. Es soll daher nur noch kurz auf einige auch für die Erfindung bedeutsame Eigenschaften und Abmessungen eingegangen werden.
Bevorzugt besitzt die erfindungsgemäße Vorrichtung demnach wenigstens eine der vorliegende Eigenschaften:
- 8 bis 30, bevorzugt 12 bis 24, insbesondere 16 bis 20 Nadeln in Umfangsrichtung der Walze,
- 2 bis 16, bevorzugt 3 bis 12 und insbesondere 4 bis 9 Nadeln pro Reihe,
- eine Überstandslänge der Nadeln über die Außenumfangsfläche von 0,1 bis 3,0 mm, bevorzugt 0,2 bis 2,5 mm und insbesondere 0,25 bis 2,0 mm,
- einen maximalen Durchmesser der Nadeln im über die Außenumfangsfläche vorstehenden Bereich von 0,05 bis 0,3 mm, bevorzugt 0,08 bis 0,2 mm,
- die Nadelenden laufen in Richtung zur Nadelspitze hin spitz zu und sind insbesondere im Bereich der Nadelspitze geschliffen,
- der Abstand zwischen den Nadelspitzen sich in zwei benachbarten Nadelreihen unmittelbar gegenüber liegender erster und zweiter Nadeln liegt zwischen 2,5 und 5 mm, bevorzugt 3,0 und 4,5 mm und insbesondere 3,5 und 4,0 mm.

Die Erfindung soll nachfolgend anhand eines Ausführungsbeispiels unter Bezugnahme auf die Zeichnung näher erläutert werden. In den Figuren, in denen gleiche Bezugszeichen gleiche Teile bezeichnen, zeigen schematisch:
- Fig. 1a: eine Seitenansicht einer erfindungsgemäßen Vorrichtung zur Behandlung der Haut;
- Fig. 1b: eine Draufsicht auf die erfindungsgemäße Vorrichtung der Figur 1 a, um 90° gedreht;
- Fig. 2a: eine Draufsicht auf eine erfindungsgemäße Vorrichtung zur Verdeutlichung der elektrischen Kontaktierung;
- Fig. 2b: die in Figur 2a gezeigte Vorrichtung um 90° gedreht;
- Fig. 3a: eine Seitenansicht auf eine Stirnseite der in Figur 2a gezeigten Walze;
- Fig. 3b: die in Figur 3a gezeigte Walze um 90° gedreht;
- Fig. 4: eine weitere Draufsicht auf die Stirnseite einer von einer Gabel gehaltenen Walze;
- Fig. 5a: eine Querschnittsansicht einer Walze;
- Fig. 5b: den in der Walze der Figur 5a verwendeten Träger in einer Querschnittsansicht;
- Fig. 5c: einen Nadelträger der Walze der Figur 5a in perspektivischer Ansicht und
- Fig. 5d: einen Nadelträger der Figur 5a im Querschnitt.

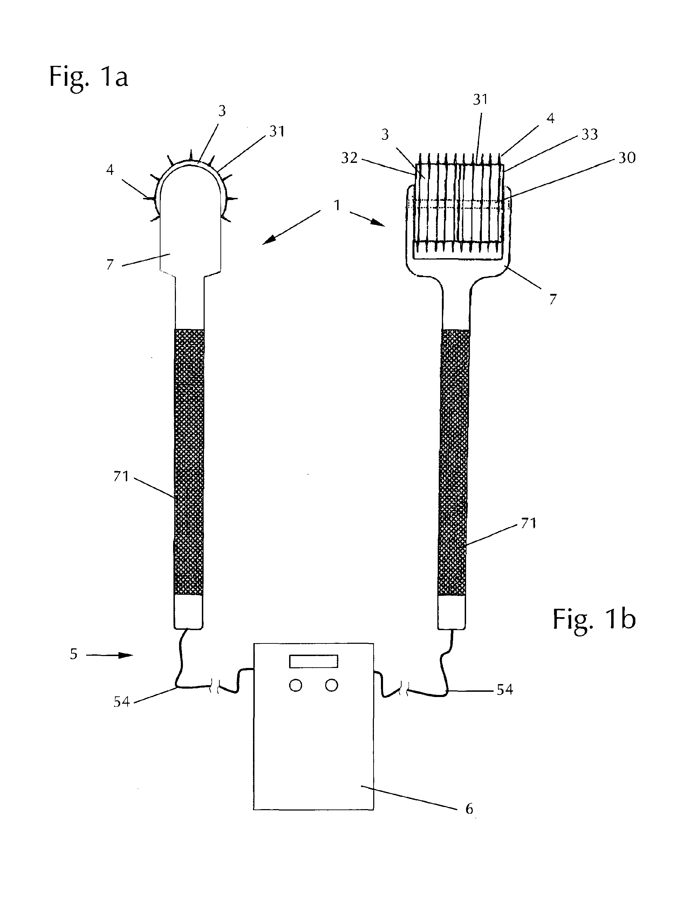

Figuren 1 a und 1 b zeigen in perspektivischer Ansicht ein Ausführungsbeispiel einer erfindungsgemäßen Vorrichtung 1 mit einem Nadelroller, der mit einer Spannungsquelle 6 verbunden ist. Der Nadelroller entspricht in seinem Grundaufbau im Wesentlichen demjenigen, der bereits aus der DE 10063634 A1 bekannt ist. Der Nadelroller weist eine Walze 3 auf, die um ihre Längsachse 30 herum an einer Gabel 7 drehbar gelagert ist. Über die Außenumfangsfläche 31 steht eine Vielzahl von Nadeln 4 radial nach außen vor. Zusätzlich zu dem aus der DE 634 bekannten Nadelroller weist die erfindungsgemäße Vorrichtung 1 Mittel 5 zum elektrischen Kontaktieren wenigstens zweier der Nadeln 4 mit der Spannungsquelle 6 auf. Die Verbindung mit der Spannungsquelle 6 erfolgt hier über ein Stromzuleitungskabel 54, das von der Walze 3 über die Gabel 7 und durch den mit der Gabel 7 verbundenen Griff 72 an dessen hinteren Ende heraus in Richtung auf die Spannungsquelle 6 geführt ist. Bei der Spannungsquelle 6 handelt es sich um eine variable Spannungsquelle, mit welcher an die kontaktierten Nadeln 4 ein linearer oder gepulster Gleichstrom angelegt werden kann. Spannung und Stromstärke können vom Benutzer entsprechend den zur Behandlung der Haut erforderlichen Bedürfnissen auf Wunsch eingestellt werden.

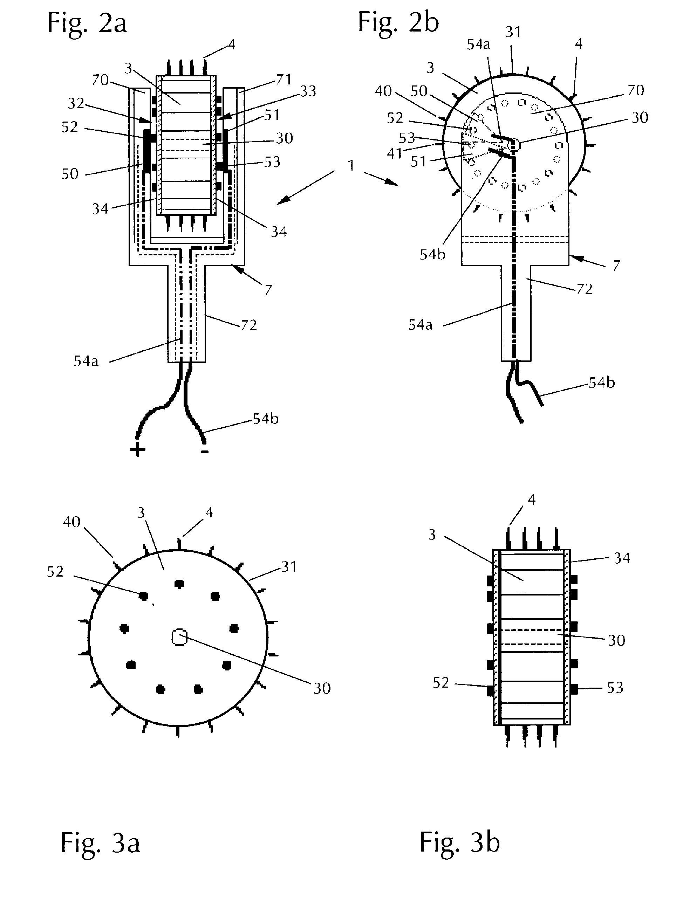

Die Art der Kontaktierung der Spannungsquelle 6 mit den Nadeln 4 ist in den nachfolgenden Figuren näher erläutert. Figur 2a zeigt dabei eine Draufsicht auf den Kopfbereich einer erfindungsgemäßen Vorrichtung 1, genauer die Draufsicht auf die Außenumfangsfläche einer Walze 3, die von einer Gabel 7 gehalten wird. Figur 2b zeigt die Vorrichtung der Figur 2a um 90° gedreht, also eine Draufsicht auf die Stirnseite der von der Gabel 7 gehaltenen Walze 3. Die elektrische Kontaktierung mit der Spannungsquelle 6 erfolgt, wie schon im Zusammenhang mit Figuren 1a und 1 b besprochen, über ein Stromzuleitungskabel 54, das in eine Zuleitung 54a, die mit dem Pluspol der Spannungsquelle 6 verbunden ist, und eine Zuleitung 54b, die mit dem Minuspol der Spannungsquelle 6 verbunden ist, aufgeteilt ist. Die beiden Zuleitungen 54a und 54b sind zunächst durch den Griff 72 der Gabel 7 geführt und teilen sich dann im gegabelten Ende auf, wobei das Kabel 54a in Richtung auf das in der Figur linke Gabelende 70 hin geführt ist, das Kabel 54b zum in der Figur rechten Ende 71 der Gabel hin. Das Kabel 54a ist im Bereich des Gabelendes 70 mit einem ersten Anschluss 50 verbunden, der zum innenseitigen Ende des Gabelbereichs 70 nach außen geführt ist und in Richtung auf die Walze 3 hin eine Kontaktfläche aufweist. Die zweite Leitung 54b ist in gleicher Weise mit einem Anschluss 51 im Bereich des rechten Gabelendes 71 verbunden.

Über den Anschluss 50 wird ein elektrisch leitender Kontakt zu einem Kontaktbereich 52 hergestellt, der über die in der Abbildung linke Stirnseite 32 der Walze 3 nach außen vorstehen. Um zu gewährleisten, dass ein ausreichend fester Kontakt zwischen Anschluss 50 und Kontaktbereich 52 entsteht, kann der Anschluss 50 federnd gelagert sein, so dass er mit ausreichendem Druck in Richtung auf den Kontaktbereich 52 gepresst wird. Bei dem Kontaktbereich 52 handelt es sich um das über die Stirnseite der Walze 3 vorstehende Ende eines Kontaktstiftes, der sich durch eine Endkappe 34, welche die Stirnseite 32 abdeckt, hindurch in die Walze 3 hinein erstreckt und dort elektrisch leitend mit einer zugehörigen Reihe von Nadeln 4 verbunden ist. Dieselbe Reihe von Nadeln ist in gleicher Weise mit einem zweiten Kontaktbereich 53 verbunden, der über die in der Abbildung rechte Stirnseite 33 der Walze 3 vorsteht. Im gezeigten Fall besteht eine Reihe aus jeweils vier Nadeln 4. Jede der insgesamt 18 Nadelreihen besitzt einen ersten und einen zweiten Kontaktbereich 52, 53. Auf die elektrische Kontaktierung von Kontaktstiften und Nadeln wird nachfolgend noch näher eingegangen werden.

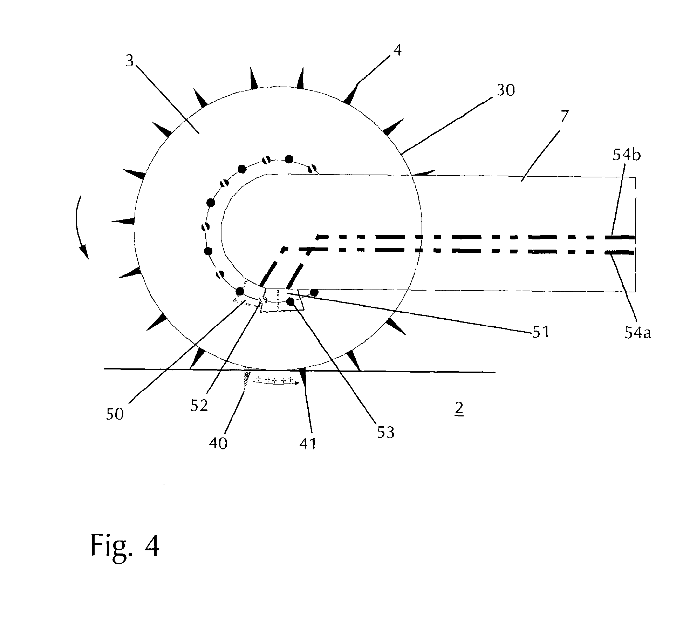

Das Herstellen und Lösen der elektrischen Kontakte zwischen Anschluss 50 und Kontaktbereich 52 einerseits sowie Anschluss 51, der ebenfalls federnd gelagert sein kann, und Kontaktbereich 53 andererseits wird durch die Rotation der Walze 3 um ihre Längsachse 30 bewirkt. Wie sich aus Figur 2b ergibt, sind die Anschlüsse 50 und 51, die sich auf gegenüberliegenden Seiten der Gabelenden 70 und 71 befinden, in Umfangsrichtung der Walze gegeneinander versetzt. Dies führt dazu, dass der erste Anschluss 50 in Kontakt mit einem ersten Kontaktbereich 52 einer ersten Reihe von Nadeln 40 kommt, der zweite Anschluss 51 dagegen mit dem zweiten Kontaktbereich 53 einer zur ersten Reihe 40 benachbarten zweiten Reihe von Nadeln 41. Die erste Reihe Nadeln 40 wird damit positiv aufgeladen, die benachbarte Reihe Nadeln 41 dagegen negativ. Die Lage der Anschlüsse 50 und 51 ist dabei so gewählt, dass die beiden kontaktierten Nadelreihen 40 und 41 beim Aufsetzen des Nadelrollers auf die Haut 2 (vergleiche Figur 4) in die Haut eindringen. Durch die unterschiedliche Polarisierung der ersten und der zweiten Nadelreihe wird in der Haut ein lonenfluss in Richtung von den positiv aufgeladenen Nadeln 40 in Richtung auf die negativ aufgeladenen Nadeln 41 induziert und so die Wundheilung durch Elektrostimulation gefördert.

Wird die Vorrichtung 1 auf der Hautoberfläche weiter vorgeschoben (in Figur 4 in Richtung der linken Blattseite), rotiert die Walze 3 dabei um die Längsachse 30 entgegen dem Uhrzeigersinn (in Pfeilrichtung). Mit der Rotation der Walze 3 bewegen sich auch die Kontaktbereiche 52 und 53 entgegen dem Uhrzeigersinn weiter und damit weg von den an der Gabel 7 gelagerten Anschlüssen 50 und 51, mit denen sie zuvor in Kontakt standen. Es soll angenommen werden, dass die Walze 3 gerade so weit gedreht wird, dass sich der erste Kontaktbereich 52 an die vorherige Position des zweiten Kontaktbereichs 53 bewegt. Damit werden die zuvor positiv aufgeladenen ersten Nadeln 40 nun über den zweiten Anschluss 51 mit dem negativen Pol der Spannungsquelle 6 verbunden und negativ aufgeladen. Sie werden somit durch das Weiterdrehen der Walze zu zweiten Nadeln 41. Die zuvor mit dem negativen Pol verbundene zweite Reihe Nadeln wird dagegen vom zweiten Kontaktbereich 53 getrennt und ist daher nicht mehr mit der Spannungsquelle 6 verbunden. In den Bereich des ersten Anschlusses 50 rückt dagegen nun die nächste - in der Figur links - benachbarte Reihe Nadeln nach, die durch Verbindung ihres Kontaktbereiches 52 mit dem Anschluss 50 jetzt positiv aufgeladen wird. In der Haut wird daher zwischen den benachbarten und unterschiedlich aufgeladenen Nadelreihenpaaren erneut ein elektromagnetisches Feld der gleichen Richtung wie das vorherige erzeugt. Die Richtung des lonenflusses in der Haut ändert sich daher durch das Vorschieben des Nadelrollers auf der Haut nicht, sondern behält Stärke und Richtung bei. Es wird also unabhängig vom Rotationsgrad der erfindungsgemäßen Vorrichtung in der Haut ein gleichbleibendes elektromagnetisches Feld erzeugt. Sollte im Verlauf einer Hautbehandlung eine Umorientierung dieses elektromagnetischen Feldes gewünscht sein, erfolgt dies durch Umstellung der Polung an der Spannungsquelle 6.

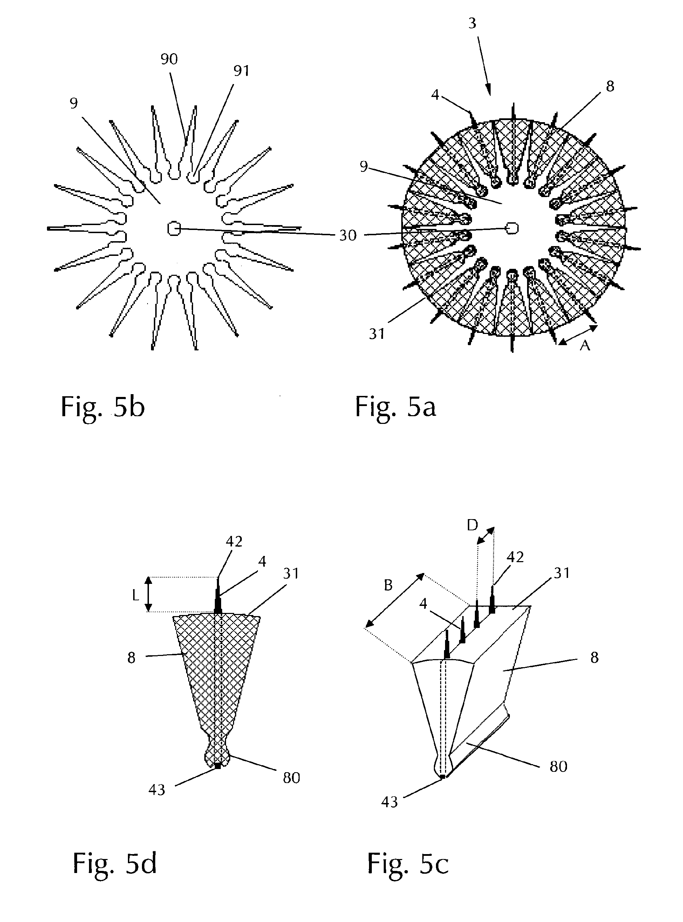

Figuren 5a bis 5d verdeutlichen den inneren Aufbau der Walze 3 und die Kontaktierung der einzelnen Nadeln mit den Kontaktbereichen 52 bzw. 53. Wie in Figur 5a erkennbar, sind die Nadeln 4 in einzelne Nadelträger 8 eingebettet, die in Schnittansicht senkrecht zur Längsachse 30 der Walze 3 einen im Wesentlichen tortenstückartigen Querschnitt aufweisen. Jeder der Nadelträger 8 verläuft im Wesentlichen über die gesamte Breite der Walze 3. Lediglich die Stirnseiten sind zusätzlich mit Endkappen 34 abgedeckt. Jeder der Nadelträger 8 nimmt eine Reihe von Nadeln 4 auf, wobei im gezeigten Fall jede Reihe aus vier einzelnen Nadeln besteht, die im gleichen Abstand D, hier etwa 2 mm, voneinander in dem Nadelträger eingebettet sind. Die Breite B des Nadelträgers 8 beträgt ca. 8 mm, mit aufgesetzten Endkappen wird eine Gesamtbreite von etwa 10 mm erreicht. Beispielsweise sind die vier Nadeln 4 einer Nadelreihe in einen Nadelträger 8 aus Kunststoff eingegossen. Ihre Spitzen stehen mit einer Länge L, beispielsweise von 0,1 bis 3 mm, über den Außenumfang 31 der Nadelträger 8 bzw. der Walze 3 vor. Die Nadeln 4 sind zumindest in diesem Vorsprungsbereich in Richtung auf die Nadelspitze 42 hin nach außen spitz zulaufend ausgebildet und vorzugsweise mit einem Schliff versehen. Der maximale Durchmesser der Nadeln 4 in ihrem über die Außenumfangsfläche 31 vorstehenden Bereich liegt zweckmäßig zwischen 0,05 und 0,3 mm, bevorzugt zwischen 0,08 und 0,2 mm. Üblicherweise wird sich der maximale Durchmesser der Nadeln in diesem Vorsprungsbereich mit der Länge L in dem unmittelbar zur Außenumfangsfläche 31 benachbarten Bereich der Nadeln 4 befinden. Der Abstand A zwischen den Nadelspitzen zweier unmittelbar benachbarter Nadelreihen beträgt im gezeigten Beispiel etwa 3,5 mm.

Im gezeigten Fall weist die Walze 3 achtzehn Nadelreihen auf und besitzt entsprechend achtzehn Nadelträger 8. Diese sind in einem Träger 9 befestigt, der im Querschnitt senkrecht zur Längsachse 30 der Walze 3 in Figur 5b dargestellt ist. Der Träger 9 besteht aus Kunststoff und hat einen im Wesentlichen sternförmigen Querschnitt mit achtzehn Strahlen 90, zwischen die jeweils die Nadelträger 8 eingepresst sind. Die Nadelträger 8 sind im Klemmsitz zwischen zwei Strahlen 90 gehalten, wobei die tropfenförmigen Fußbereiche 80 in entsprechende kugelförmige Ausnahmen 91 im Träger 9 eingreifen, eine Art Rastverbindung bilden und so für einen sicheren Sitz der Nadelträger 8 im Träger 9 sorgen. Falls gewünscht, können die Nadelträger 8 auch im Träger 9 eingeklebt werden.

Für die elektrische Kontaktierung der in einem Nadelträger 8 befestigten Nadeln einer Nadelreihe gibt es verschiedene Möglichkeiten. Eine besteht darin, die Nadeln im Bereich ihrer Fußpunkte 43 mittels eines leitfähigen Klebstoffes, eines Kabels oder in ähnlicher Weise miteinander zu verbinden. Hier wurde jedoch die Möglichkeit gewählt, das Material des Nadelträgers 8 leitfähig zu machen. Dabei kann es grundsätzlich ausreichend sein, lediglich einen Teilbereich des Nadelträgers 8, beispielsweise den Fußbereich 80, aus dem leitfähigen Material zu fertigen. Im gezeigten Fall ist jedoch der Nadelträger insgesamt aus einem leitfähigen Kunststoff hergestellt, der für die elektrische Kontaktierung sämtlicher Nadeln untereinander und die elektrisch leitende Verbindung zu den zugehörigen Kontaktbereichen 52 und 53 sorgt. Die Kontaktbereiche 52 und 53, die sich, wie beispielsweise Figur 3b zu entnehmen ist, an unterschiedlichen Stirnseiten eines jeden Nadelträgers 8 befinden, werden hier durch Kontaktnippel gebildet, die durch passende Bohrungen in den Endkappen 34 geführt sind und mit ihren innenseitigen Enden an einer jeweiligen Stirnseite des Nadelträgers 8, beispielsweise durch Kleben, befestigt und mit dem elektrisch leitfähigen Nadelträger 8 und/oder den anderweitig untereinander kontaktierten Nadeln leitend verbunden sind. Wie vorstehend bereits beschrieben, kommt - abhängig von der Rotation der Walze 3 um ihre Längsachse 30 - entweder der erste Kontaktbereich 52 mit dem ersten Anschluss 50 in elektrisch leitenden Kontakt oder der zweite Kontaktbereich 53 mit dem zweiten Anschluss 51. Dadurch werden die Nadeln 4 entweder mit dem positiven Pol oder dem negativen Pol der Spannungsquelle 6 verbunden und entsprechend aufgeladen. Kurzschlüsse zwischen den benachbarten Nadelträgern 8 und den zueinander benachbarten Nadelreihen werden dadurch vermieden, dass das Material des Trägers 9 nichtleitend ist und so die einzelnen Nadelträger 8 gegeneinander isoliert werden, selbst wenn diese insgesamt aus einem leitfähigen Material bestehen.

Literatur:
1. Foulds L., Barker A. Human skin battery potentials and their possible role in wound healing. Br J Dermatol 1983;109:515-22.
2. L. C. Kloth, Electrical Stimulation for Wound Healing: A Review of Evidence From In Vitro Studies, Animal Experiments, and Clinical Trials. 2005 Sage Publications.
3. Jaffe L., Vanable J. Electrical fields and wound healing. Clin Dermatol 1984;2(3):34-44.
4. Cheng K., Tarjan P., Oliveira-Gandia M., et al. Anocclusive dressing can sustain natural electrical potential of wounds. J Invest Dermatol 1995;104(4):662-5.
5. Eltinge E., Cragoe E. Jr., Vanable J. Jr. Effects of amiloride analogues on adult Notophthalmus viridescens limb stump currents.Comp Biochem Physiol 1986;89A:39-44.
6. Falanga V., Bourguignon G., Bourguignon L. Electrical stimulation increases the expression of fibroblast receptors for transforming growth factor-beta. J Invest Dermatol 1987;88:488-92.
7. Orida N., Feldman J. Directional protrusive pseudopodial activity andmotility in macrophages induced by extra-cellular electric fields. Cell Motil 1982;2:243-55.
8. Monguio J. Über die polare Wirkung des galvanischen Stromes auf Leukozyten. Z Biol 1933;93:553-9.
9. Fukushima K., Senda N., Inui H., et al. Studies of galvanotaxis of leukocytes. Med J Osaka Univ 1953;4(2-3):195-208.
10. Dineur E. Note sur la sensibilities des leukocytes a l'electricite. Bulletin Seances Soc Belge Microscopic (Bruxelles) 1891;18:113-8.
11. Canaday D., Lee R. Scientific basis for clinical application of electric fields in soft tissue repair. In: Brighton C, Pollack S, editors. Electromagnetics in biology and medicine. San Francisco: San Francisco Press; 1991.
12. Erickson C., Nuccitelli R. Embryonic fibroblastmotility and orientation can be influenced by physiological electric fields. J Cell Biol 1984;98:296-307.
13. Yang W., Onuma E., Hui S. Response of C3H/10T1/2 fibroblasts to an external steady electric field stimulation. Exp Cell Res 1984;155:92-7.
14. Nishimura K., Isseroff R., Nuccitelli R. Human keratinocytes migrate to the negative pole in direct current electric fields comparable to those measured in mammalian wounds. J Cell Sei 1996;109:199-207.
15. Sheridan D., Isseroff R., Nuccitelli R. Imposition of a physiologic DC electric field alters the migratory response of human keratinocytes on extracellular matrix molecules. J Invest Dermatol 1996;106(4):642-6.
16. Min Zaho. Electrical fields in wound healing-An overriding signal that directs cell migration. Elsevier 2008, page 674 - 680.
17. Min Zhao et al. Electrical signals control wound healing through phosphatidylinositol-3-OH kinase-Y and PTEN. Nature 2006, Vol 442/27.
18. Fang K., lonides E., Oster G., et al. Epidermal growth factor receptor relocalization and kinase activity are necessary for directional migration of keratinocytes in DC electric fields. J Cell Sci 1999;112:1967-78.
19. Rowley B. Electrical current effects on E. coli growth rates. Proc Soc Exp Biol Med 1972;139:929-34.
20. Wolcott L., Wheeler P., Hardwicke H., et al. Accelerated healing of skin ulcers by electrotherapy: preliminary clinical results. South Med J 1969;62:795-801.
21. Deitch E., Marino A., Malakanok V., et al. Electrical augmentation of the antibacterial activity of silver nylon. Proceedings of the 3rd Annual BRAGS; 1983 Oct 2-5; San Francisco.
22. Deitch E., Marino A., Gillespie T., et al. Silver nylon: a new antimicrobial agent. Antimicrobial Agents Chemother 1983;23:356-9.
23. Marino A., Deitch E., Albright J. Electric silver antisepsis. IEEE Trans Biomed Eng 1985;32(5):336-7.
24. Colmano G., Edwards S., Barranco S. Activation of antibacterial silver coatings on surgical implants by direct current: preliminary studies in rabbits. Am J Vet Res 1980;41 (6):964-6.
25. Thibodeau E., Handelman S., Marquis R. Inhibition and killing of oral bacteria by silver ions generatedwith lowintensity direct current. J Dent Res 1978;57:922-6.
26. Alvarez O., Mertz P., Smerbeck R., et al. The healing of superficial skin wounds is stimulated by external electrical current. J Invest Dermatol 1983;81 (2):144-8.
27. Falcone A., Spadero J. Inhibitory effects of electrically activated silver material on cutaneous wound bacteria. Plast Reconstr Surg 1986;77(3):445-58.
28. Becker R., Spadero J. Treatment of orthopedic infections with electrically generated silver ions. J Bone Joint Surg Am 1978;60(7):871-81.
29. Junger M., Zuder D., Steins A., et al. Treatment of venous ulcers with low frequency pulsed current (Dermapulse): effects on cutaneous microcirculation. Der Hautarzt 1997;18:879-903.

## Patentansprüche

1. Vorrichtung (1) zur Behandlung der Haut (2) mit einer um ihre Längsachse (30) drehbar gelagerten Walze (3), auf deren Außenumfangsfläche (31) eine Vielzahl von Nadeln (4) radial nach außen vorsteht, und welche Mittel (5) zum elektrischen Kontaktieren wenigstens eines Teils der Nadeln (4) mit einer Spannungsquelle (6) aufweist, umfassend
- einen ersten, mit einem ersten Pol der Spannungsquelle (6) verbindbaren Anschluss (50),
- einen zweiten, mit einem zweiten Pol der Spannungsquelle (6) verbindbaren Anschluss (51),
- einen ersten Kontaktbereich (52), der elektrisch leitend mit ersten Nadeln (40) verbunden ist,
- einen zweiten, vom ersten Kontaktbereich (52) räumlich getrennten und elektrisch isolierten zweiten Kontaktbereich (53), der elektrisch leitend mit zweiten Nadeln (41) verbunden ist,
wobei die Anschlüsse (50, 51) und die Kontaktbereiche (52, 53) so ausgebildet sind, dass bei einer Verbindung von erstem Anschluss (50) und erstem Kontaktbereich (52) auch der zweite Anschluss (51) mit dem zweiten Kontaktbereich (53) verbunden ist, und
worin die ersten Nadeln (40) und die zweiten Nadeln (41) jeweils in einer Reihe angeordnet und die Reihen in Umfangsrichtung der Walze (3) voneinander beabstandet sind.

2. Vorrichtung (1) nach Anspruch 1,
worin erster und zweiter Kontaktbereich (52, 53) zu einer Oberfläche der Walze (3) herausgeführt sind, insbesondere zu einer Stirnseite, und sich bevorzugt auf gegenüberliegenden Stirnseiten (32, 33) der Walze (3) befinden.

3. Vorrichtung (1) nach Anspruch 2,
worin erster und zweiter Anschluss (50, 51) an einem innenseitigen Ende und insbesondere an sich gegenüberliegenden Enden (70, 71) einer die Walze (3) haltenden Gabel (7) angeordnet sind.

4. Vorrichtung (1) nach einem der Ansprüche 1 bis 3,
worin erster Anschluss (50) und erster Kontaktbereich (52) sowie zweiter Anschluss (51) und zweiter Kontaktbereich (53) jeweils lösbar miteinander verbunden sind, wobei die jeweilige Verbindung bevorzugt durch Rotation der Walze (3) um ihre Längsachse (30) herstellbar oder trennbar ist.

5. Vorrichtung (1) nach Anspruch 4,
worin Anschlüsse (50, 51) und Kontaktbereiche (52, 53) als Schleifkontakte ausgebildet sind.

6. Vorrichtung (1) nach einem der Ansprüche 1 bis 5,
worin die ersten Nadeln (40) und die zweiten Nadeln (41) jeweils in einer zur Längsachse (30) der Walze (3) parallel verlaufenden Reihe angeordnet sind.

7. Vorrichtung (1) nach Anspruch 6,
worin die Reihen von Nadeln (40; 41) jeweils in einen Nadelträger (8), insbesondere einen im Wesentlichen tortenstückförmigen Nadelträger, eingebettet sind, welcher bevorzugt zumindest teilweise aus einem leitfähigen Kunststoff besteht.

8. Vorrichtung (1) nach Anspruch 7,
worin die Nadelträger (8) von einem Träger (9) aus einem nichtleitenden Material, insbesondere Kunststoff, mit einem im Wesentlichen sternförmigen Querschnitt in Richtung senkrecht zur Längsachse (30) der Walze (3) gehalten werden, der die Nadelträger (8) elektrisch gegeneinander isoliert.

9. Vorrichtung (1) nach einem der Ansprüche 5 bis 8,
worin eine Vielzahl von Nadelreihen in Umfangsrichtung der Walze (3) zueinander benachbart angeordnet ist, welche sowohl einen ersten Kontaktbereich (52) als auch einen zweiten Kontaktbereich (53) aufweisen, bevorzugt an gegenüberliegenden Enden der Nadelreihen.

10. Vorrichtung (1) nach einem der Ansprüche 5 bis 9,
worin die Anschlüsse (50, 51) so ausgebildet sind, dass in Umfangsrichtung der Walze (3) zueinander unmittelbar benachbarte Reihen von Nadeln (40; 41) elektrisch mit der Spannungsquelle (6) kontaktierbar sind.

11. Vorrichtung (1) nach einem der Ansprüche 1 bis 10,
worin sie wenigstens eine der folgenden Eigenschaften besitzt:
- 8 bis 30, bevorzugt 12 bis 24, insbesondere 16 bis 20 Nadeln in Umfangsrichtung der Walze (3),
- 2 bis 16, bevorzugt 3 bis 12 und insbesondere 4 bis 9 Nadeln pro Reihe,
- eine Überstandslänge (L) der Nadeln über die Außenumfangsfläche (31) von 0,1 bis 3,0 mm, bevorzugt 0,2 bis 2,5 mm und insbesondere 0,25 bis 2,0 mm,
- einen maximalen Durchmesser der Nadeln im über die Außenumfangsfläche (31) vorstehenden Bereich von 0,05 bis 0,3 mm, bevorzugt 0,08 bis 0,2 mm,
- die Nadelenden laufen in Richtung zur Nadelspitze (42) hin spitz zu und sind insbesondere im Bereich der Nadelspitze geschliffen,
- der Abstand (A) zwischen den Nadelspitzen (42) sich in zwei benachbarten Nadelreihen unmittelbar gegenüber liegender erster und zweiter Nadeln (40, 41) liegt zwischen 2,5 und 5 mm, bevorzugt 3,0 und 4,5 mm und insbesondere 3,5 und 4,0 mm.

12. Vorrichtung (1) nach einem der Ansprüche 1 bis 11,
worin die Spannungsquelle (6) ausgebildet ist, eine Spannung, insbesondere eine lineare oder gepulste Gleichspannung, zuzuführen, und bevorzugt als variable Spannungsquelle ausgebildet ist.
